# EUROPEAN PATENT APPLICATION

(11) **EP 4 821 839 A1**
(43) Date of publication of application: **14.10.2026**
(21) Application number: 25169911.2
(22) Date of filing: 10.04.2025
(51) Int. Cl.: B65D 21/08, A61J 1/16, A61M 5/00, B65D 33/02, B65D 77/04, B65D 81/20

(54) **MEDICAL COMPONENT CONTAINER**

(71) Applicant: Novo Nordisk A/S, 2880 Bagsvaerd (DK)
(72) Inventor: VUJOVITS, András, 2880 Bagsværd (DK); JAKOBSEN, Nikolaj Eusebius, 2880 Bagsværd (DK); KRONBORG, Nikolaj Egdal, 2880 Bagsværd (DK)

(57) **Abstract**

A medical component container (200) comprising: a) a container body (100) having a base (102), a peripheral wall (107) having a lower end that is contiguous to a perimeter of the base (102) and extending upward towards peripheral edges surrounding and opening, and a reinforcing frame (106) that surrounds the opening, b) a protective film (150) that is attached to the peripheral edges for sealing the opening, amd c) two or more vertically stacked trays (10) each holding a plurality of medical components (1) in a regular arrangement, the lowermost tray (10) being supported on or at the base (102), wherein the peripheral wall (107) is configured for collapsing so that, after the protecting film (150) has been removed from the peripheral edges, and upon an external force acting to move the base (102) vertically upwards, the stacked trays (10) are lifted by the base (102) to emerge upwards from the plurality of peripheral edges. Also disclosed is a medical component container (200) having a protecting film (150) removable by an automatic gripper.

## Description

The present disclosure generally relates to pharma production components, such as pharmaceutical products and medical devices and, more particularly, systems for packaging, storing and handling medical components by way of sterilized medicament component containers.

### BACKGROUND OF THE INVENTION

Within pharmaceutical product manufacturing, much effort is directed into improving efficiency, capacity and quality, and automation and subcomponent handling by automated handling are important steps for obtaining these goals.

As an example, within medical filling plants, component containers are typically used for storing and handling a plurality of medical components, such as medication containers, bottles and closure members in a structured way, such accommodated in rigid structures provided in tubs or nests.

Various component containers for medical trays, such as tubs and nests have been suggested in the art, including references WO 2018/20505 A1 and US 2021053313 A.

Component containers may in an organized way accommodate a single or a plurality of stacked trays within a closed sterility barrier, such as an aseptic packaging, typically made from plastic bags. One of the bottlenecks in manufacturing and filling lines is the debagging process where sterilized filled trays, such as tubs and/or nests accommodating a large number of components are being fed into the line. In the debagging process aseptic layers are removed to transfer the trays into an adjacent production class. In order to increase line output and keep up with the growing demand an important object is to find a faster way to unpack trays/nests and feed them into the filling lines.

Having regard to the above, it is an object of the present invention to provide a medical component container which enables faster and safer unpacking while avoiding compromising of sterility. A further object of the invention is to provide a medical component container which offers more efficient removal and subsequent handling of trays accommodated within a medical component container. A further object of the invention is to provide a medical component container design which is improved with regard to obtaining a space-saving solution and which offers improvements with regard to reducing the need of materials bound for discarding.

### DISCLOSURE OF THE INVENTION

In the disclosure of the present invention, embodiments and aspects will be described which will address one or more of the above objects or which will address objects apparent from the below disclosure as well as from the description of exemplary embodiments.

In a first aspect of the invention a medical component container is provided, comprising:
- a container body having a substantially rectangular base, a peripheral wall having a lower end that is contiguous to a perimeter of the base and extending upward, and a plurality of peripheral edges being contiguous to an upper end of the peripheral wall wherein the container body has an opening surrounded by the plurality of peripheral edges, and wherein the plurality of peripheral edges of the container body further comprises a reinforcing frame that surrounds the opening and provides structural reinforcement for the plurality of peripheral edges,
- a protecting film that is attached, such as by being adhered or heat-sealed, to the plurality of peripheral edges for covering and sealing the opening,
- either a single tray or two or more vertically stacked trays, each tray being substantially rectangular and each holding a plurality of medical components in a regular arrangement, the lowermost tray being supported on or at the base,
wherein the peripheral wall is configured for collapsing in a manner wherein the base is movable upwards towards the opening so that, after the protecting film has been removed from the peripheral edges, and upon an external force acting to move the base vertically upwards, the tray/stacked trays are lifted by the base to emerge upwards from the plurality of peripheral edges.

In accordance herewith, the medical component container enables use of an automated container-handling system capable of unloading a single or multiple trays (nests and or tubs) without mechanically reaching through different sterility grades or causing second air contamination within the containers volume. The trays may be lifted and transferred from the container into grade C environment. During this lifting operation an actuator pushes on the soft bottom of the container body and folds the sidewalls as the lifting movement occurs, so the lifting happens without the actuator directly touching the trays. When the unloading of trays stops it is possible to partially close the opening to reduce the load on an associated HVAC system.

In some variants the reinforcing frame is attached to the plurality of peripheral edges.

In some variants thereof, the peripheral wall is made of a first material having a first rigidity, and wherein the reinforcing frame is made from a second material having a second rigidity greater than the first rigidity.

In other variants, the reinforcing frame is formed unitarily with the peripheral wall as a thickened portion. Hence, when the reinforcing frame and the peripheral wall is formed as a unitary member the reinforcing frame is provided as structural reinforcing element contiguous to the peripheral wall.

In some embodiments, the plurality of peripheral edges comprise material portions being contiguous to an upper end of the peripheral wall and extend outwardly to define a contiguous flange portion, and wherein the protecting film is sealingly attached, such as being adhered or heat-sealed to the contiguous flange portion.

In some variants the peripheral wall comprises four side walls configured as pairs of side walls arranged in opposing manner, wherein each side wall comprises a plurality of vertically stacked horizontally extending panels being interconnected by horizontally extending living hinge portions.

In some variants, the peripheral wall I is configured to assume a corrugated shape when the container body assumes a collapsed state. Hence, as the base is being lifted, the trays will emerge from the top of the container enabling easy removal of the trays.

In a second aspect of the invention, a medical component container is provided, comprising-
a container body having a substantially rectangular base, a peripheral wall having a lower end that is contiguous to a perimeter of the base and extending upward, and a plurality of peripheral edges being contiguous to an upper end of the wall and extending outwardly wherein the container body has an opening surrounded by the plurality of peripheral edges, the plurality of peripheral edges defining first and second edges arranged in parallel and opposite to each other and third and fourth edges opposite to each other, the third and fourth edges extending transversely and interconnecting the first and second edges,
- a protecting film having a first film section that is adhered or heat-sealed to the plurality of peripheral edges for covering and sealing the opening, wherein the protecting film has a second film section at least partially overlapping the first film section, the second film section having a gripping portion adjacent said first edge, and being attached to the first film section along said second edge so that, when the protecting film is peeled open by exertion of an external pulling force on the gripping portion in a direction away from the second edge, the first film section separates initially from the plurality of peripheral edges at a portion disposed at the second edge and progressing gradually towards the first edge.

In accordance herewith, to prevent contact between the sterility grades during automated handling of the medical container component, extra material has been added to the protecting film of the container, allowing the film to fold over itself. This overfolded top layer can be grabbed from the side by an automated gripper, which ensures that all moving parts remain below the container opening while it is being opened. The automated gripper then moves to the side to peel back the film of the container.

In some embodiments the opening of the container body is rectangular, such as substantially rectangular.

In some applications the said external pulling force is either exerted laterally away outwards from the second edge or exerted at an inclined angle relative to the lateral direction away outwards from the second edge, enabling initial peeling at a corner section of the flange.

In some other applications said external pulling force is exerted in a direction either transverse to the first edge or generally diagonally away from the opening.

In some embodiments, the plurality of peripheral edges surrounding the opening of the container body extend within a plane and wherein the external pulling force either exerts a force in a direction within the plane or in a direction forming an angle of inclination relative to said plane, either upwards or downwards.

In some variants, the second film section lies flat on top of the first film section.

Also, in some variants, the second film section and the first film section are either made from single-sheet material and folded along the second edge, or alternatively, wherein the second film section is attached to the first film section along said second edge or at discrete portions along the second edge.

In some embodiments, the first edge comprises one or more flange sections contiguous to an upper end of the wall and extending laterally outward therefrom, wherein the second film section adheres to at least a portion of the flange sections, optionally via the first film section, towards a free, non-adhered, gripping area of the second film section.

Furthermore, the container may be provided in forms wherein the container accommodates two or more vertically stacked trays, each tray being substantially rectangular and each holding a plurality of medical components in a regular arrangement, the lowermost tray being supported on or at the base,
wherein the peripheral wall is configured for collapsing in a manner wherein the base is movable upwards towards the opening so that, after the protecting film has been removed from the peripheral edges, and upon an external force acting to move the base vertically upwards, the stacked trays are lifted by the base to emerge upwards from the plurality of peripheral edges.

In some forms the protecting film is a gas-permeable plastic film enabling sterilization of the interior of the medical component container through the protecting film by a flow of a sterilization gas, such as ethylene oxide, or by steam sterilization.

Further beneficial embodiments according to the second aspect may include any of the options listed above in connection with the embodiments according to the first aspect.

### BRIEF DESCRIPTION OF THE DRAWINGS

In the following embodiments of the invention will be described with reference to the drawings, wherein:
Fig. 1 shows a schematic perspective representation of an exemplary embodiment of a material in-feed station serving debagging and unloading of nested products,
figs. 2a-2d shows different views of a first embodiment of a medical component container according to the invention,
figs. 3a and 3b shows a second embodiment of a medical component container prior to and after removing a protecting film seal, respectively,
fig. 4a shows the material in-feed station of fig. 1 wherein a medical component container has been received by a conveyer belt in CNC area,
fig. 4b is a similar view but where the full and closed container has arrived in reference position underneath the opening to class C,
fig. 5a is a top view of the in-feed station focussing on the debagging area where a protecting film has been initially peeled off from the container,
fig. 5b is a top view of the debagging area where the protecting film has been almost entirely detached from the container,
fig. 6 is a perspective view of the in-feed station wherein the protecting film has been removed and bottom lifters has pushed the opened container with stacked nests against the opening to class C,
fig. 7 is a view wherein the bottom lifters of fig. 6 have pushed the base of the container for lifting the nests of the container into class C, and
fig. 8, is a view wherein the uppermost nest of the container has been moved from the stack of nests towards the next station.

In the figures like structures are mainly identified by like reference numerals.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation during production. The term "distal" refers to a portion of an element, assembly or device which during use is oriented towards a skin surface of a user, the term "proximal" referring to the opposed portion. Correspondingly, for a traditional cartridge configured for needle injection, the needle end of the cartridge is arranged at the distal end and, typically, the plunger stopper is initially positioned in a proximal end of the cartridge barrel. The shown figures are schematic representations for which reason the configuration of the different structures as well as their relative dimensions are intended to serve illustrative purposes only. When the term member or element is used for a given component, it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

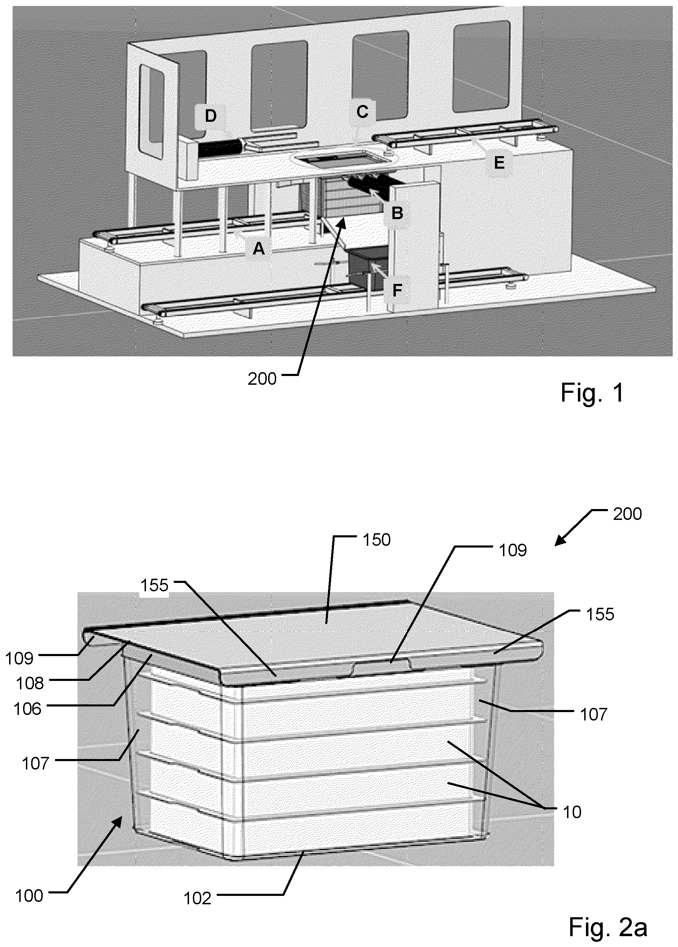

Referring to fig. 1, an exemplary embodiment of a material in-feed station in a production line is shown, the material in-feed station serving debagging and unloading of products being held in nests and/or tubs. The shown station is configured for receiving closed and sealed medical component containers 200 each accommodating nested products in a stacked configuration, optionally including tubs for accommodating the products or for grouping or protecting nest configurations. A non-limiting example of nested products may be nested products arranged in columns and rows in each nest, e.g. products such as empty sterilized cartridge containers 1 each comprising a glass barrel and being pre-crimped with a penetrable membrane at a shoulder section of the barrel. The labels in fig. 1 refers to the following. "A": conveyer belt in CNC class; "B": gripper to remove protecting film (Tyvek); "C": opening connecting class CNC and C; "D": pusher to push nest on conveyer belt; "E": conveyer belt in class C; "F": waste container to store folded containers and peeled off Tyvek.

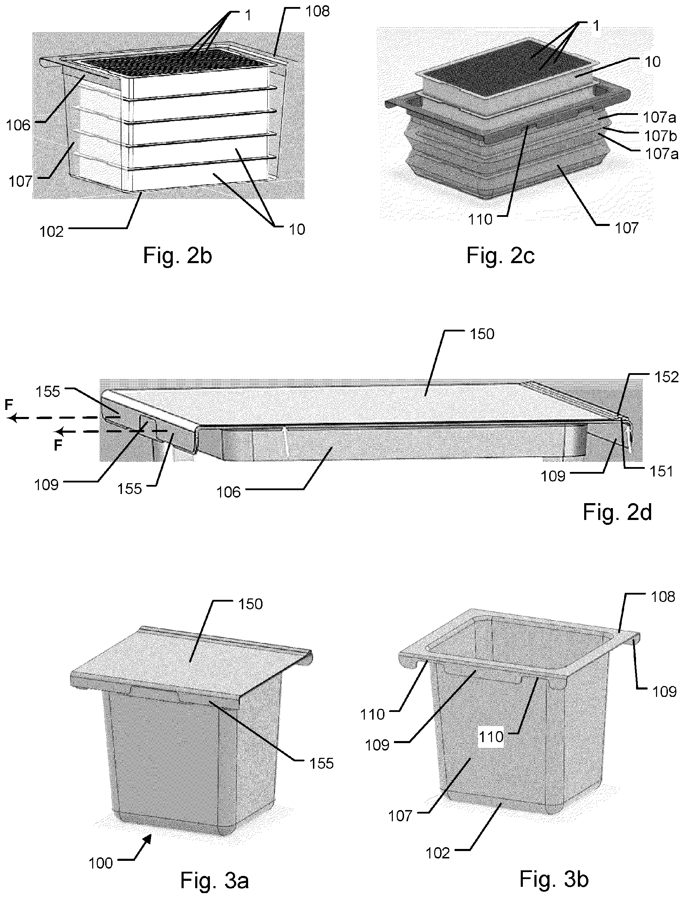

In the following, mainly referring to figs. 2a-2d, a container 200 in a first embodiment according to the invention will be described. The container 200 comprises a container body, i.e. an outer packaging, generally referred to by reference 100, and will in its finished form provide space for a plurality of stacked component nests 10 to be accommodated within its interior. The outer packaging 100 comprises a soft (or semi-rigid) bag having a rectangular base 102 and four upwards extending sidewalls 107 each ending at the top of the outer packaging 100, thus defining peripheral edges that surrounds a rectangular opening located at the top. Figure 2 offers a semi-transparent view enabling five stacked nests 10 to be viewed through the sidewalls 107. A rigid closed-loop frame 106 encircles the opening and structurally reinforces the sidewalls 107 at their peripheral edge. The soft bag is welded to the rigid frame or may alternatively be fastened by other means. A protecting film 150 provided as a lid (made from Tyvek) is glued to the frame and thereby forms an aseptic seal of the opening of the outer packaging 100.

The frame 106 is in the embodiment shaped so as to exhibit an outward extending flange 108. The outwards extending flange, at two opposite sides, curve downwardly to a vertically downwards extending gripping portion 109. One of the two gripping portions 109 has two cut-outs 110 each forming a notch defined therein (see fig. 2c), thereby leaving a central gripping area between the notches. The Tyvek lid 150 is glued to the frame 106 along the perimeter and folded at a side edge along a fold edge so as to be superposed the first layer (see fig. 2d at the right-hand side). The overfolded layer is secured at the opposite side edge in place with a drop of glue. The lid features a small central cutout that allows the rigid frame (gripping portion 109) to be gripped and secured while the lid 150 is being pulled. For this purpose, the lid includes two flaps 155 each overlapping a respective one of the two cut-outs 110. As the flaps 155 remains un-supported at the notches, the flaps can be easily gripped and pulled, such as by a lid gripping arm of the in-feed station. Hence, the flaps form two pull-tabs which enables pulling of the lid when an external force F is applied, such as in a direction indicated in fig. 2d.

In the shown embodiment, the lid 150 is formed from a single-sheet film material comprising a first layer 151 being sealingly attached to the flange 108 along the entire perimeter thereof, and folded to form a second layer 152 which extend on top of the layer all the way to the gipping portion, i.e. where the second layer forms two pull-tabs 155. The second layer exclusively serve the purpose of forming a mechanical pull section allowing the force exerted on the pull tabs to be transferred to the fold line, thereby serving to gradually separate the fist layer 151 from the flange 108, initially from the right-hand side in fig. 2d and gradually towards the left-hand most areas of flange 108. It is to be noted that the force for pulling the lid need not occur in direction within the plane of the opening but may in other embodiments include pulling at an angle above or below the horizontal direction. Also, in some embodiments, the pull force may be applied in the plane but in directions aligned with a diagonal direction so as to provide an initial peeling at one corner of the flange rather than at the entire side edge where the two foil areas 151 and 152 are attached to each other. Smaller area of peeling generally results in smaller and more predictable and even forces and lead to a more controlled process.

It is to be noted that the first layer 151 and or the second layer may be made of the same material or different materials, possibly formed from layered products. It is also to be noted that the second layer 152 need not necessarily form a continuous layer omitting any holes or openings, as the primary function of the second layer 152 serves as a member transferring forces for pulling the first layer at the correct location, generally oppositely disposed from the location of the pull tabs 155. For example, the second layer 152 may form one or more narrow bands significantly narrower than the width of the first layer 151 or may form a full-width band with one or more ventilation openings.

Figs. 3a and 3b shows a second embodiment outer packaging 100, the figures providing additional details of the lid 150 and the frame design of the medical component container 200.

Turning now to fig. 2c a different aspect of the medical component container 200 is illustrated. As the sidewalls 107 of the outer packaging 100 is formed by soft pliable material, such as a polyethylene or polypropylene resin, the sidewalls 107 form collapsible wall portions. Hence, after removal of the lid 150, and upon exertion of a push force directed vertically upwards on the base 102, possibly simultaneously retaining the frame 106 at a fixed position, the base of the outer packaging starts to move upwards thereby lifting the stacked nests 10 while the sidewalls 107 collapse. This is indicated in fig. 2c, and the effect is utilized to enable the nests 10, on-by-one, being gripable by a handling actuator for removal from the outer packaging 100.

Unpredictable creasing of the sidewalls 107 could theoretically block the nests 10 from leaving the outer package 100. To solve this potential issue the soft bottom of the outer package 100 may be produced with a pattern that creates predictable folding. In the shown example, the sidewalls each comprises a plurality of vertically stacked horizontally extending panels 107a being interconnected by horizontally extending living hinge portions 107b. Hence, the sidewalls 107 is configured to assume a corrugated shape when the outer packaging 100 assumes a collapsed state. An example of this is shown in fig. 2c, where the Tyvek lid 150 has been removed and the nests 10 are being unloaded successively through the opening of the outer packaging 100, i.e. one after the other.

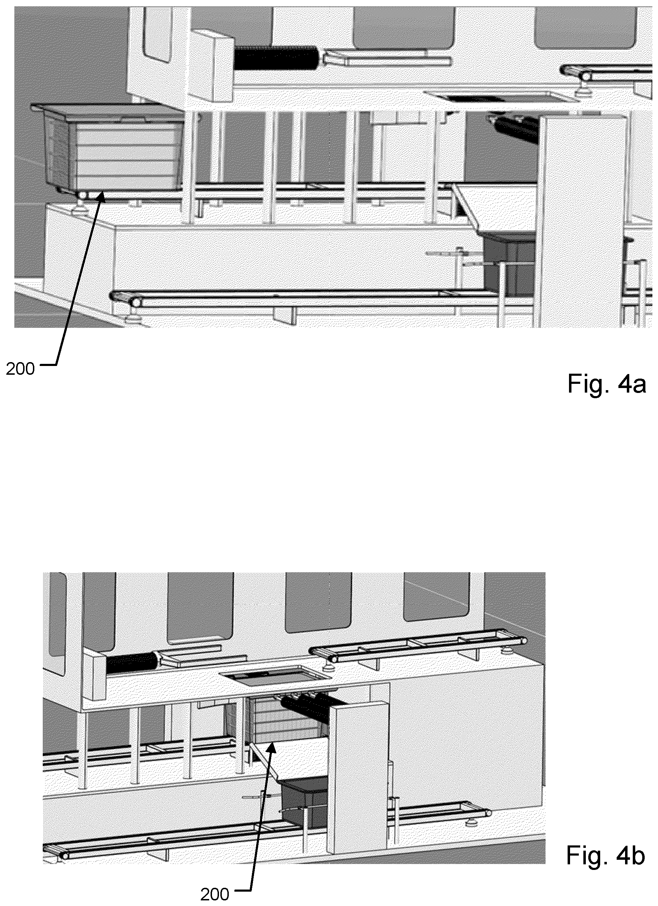

While referring generally to fig. 1 described above, the "debagging procedure" and the nest removal procedure will next be described with reference to figs. 4a through 8.

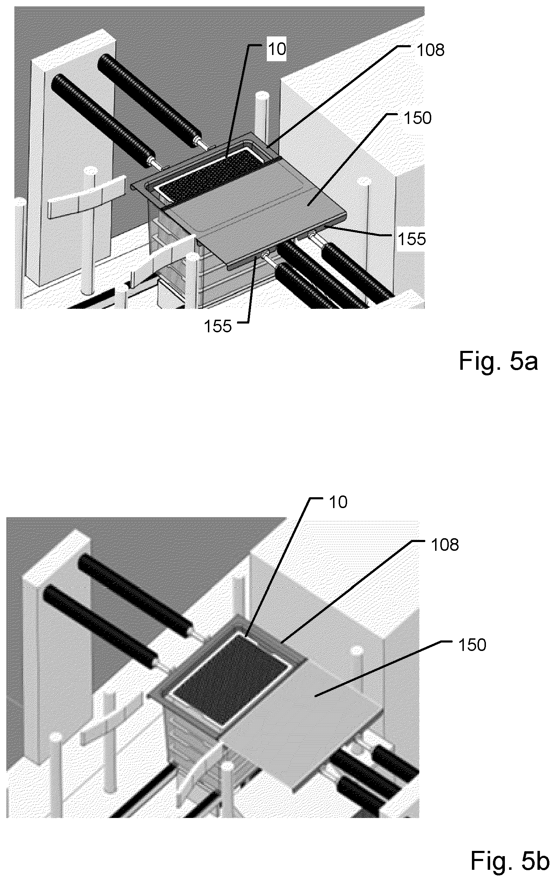

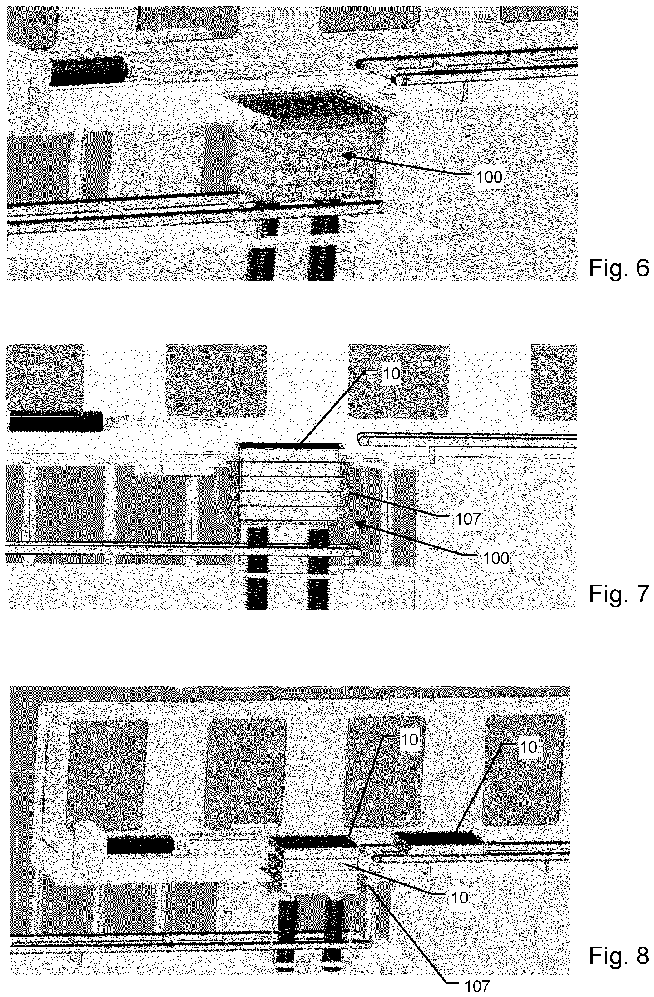

The de-bagger module of the station is situated partially in grade CNC and partially in grade C. It receives the containers 200 from a de-palletizer module via a conveyor belt and outputs product nests/tubs which proceed into an electron beam tunnel. Designed to operate autonomously, the module executes the following steps:
1. Transport the container 200 containing stacked nests 10 from the de-palletizer interface (fig. 4a).
2. Orient the container 200 using passive guiding features to ensure that the long side is parallel to the direction of travel.
3. Stop the container 200 under the opening leading to grade C (fig. 4b).
4. Grip the rigid frame 106 to secure the container 200.
5. Grip the overfolded end of the lid 155 (fig. 5a)
6. Peel away and discard the lid 155 (figs. 5a and 5b).
7. Lift the stack of nests 10 by pushing through the soft bottom (fig. 7).
8. In the grade C section, catch the topmost nest 10 at its brim (fig. 8).
9. Discard the empty "bag" 102/107/106.
10. Transport and place the unloaded nest 10 onto the exit conveyor (fig. 8).
11. Convey the unloaded nests 10 to the next module, the electron beam tunnel.

### Aseptic Design Considerations

The main requirement for consideration is to create a bag-handling system capable of unloading multiple nests/tubs without mechanically reaching through different grades or causing second air contamination within the bag's volume. To prevent contact between the grades, extra material was added to the lid of the bag, allowing it to fold over itself. This overfolded top layer can be grabbed from the side by an automated gripper, which ensures that all moving parts remain below the bag opening while it is being opened. The gripper then moves to the side to peel back the lid of the bag. The opening of the bag occurs under an opening leading to grade C, where air from grade C is directed onto the bag, protecting the nests/tubs from CNC air. For this to happen reliably the frame of the bag must be close to the plane of the opening. The tubs are lifted and transferred from the bag into grade C environment. During this lifting operation an actuator pushes on the soft bottom of the bag and folds it in synchronicity with the lifting movement, so the lifting happens without the actuator directly touching the tubs. When the unloading of nests/tubs stops it is possible to partially close the opening to reduce the load on the HVAC system.

### Up-classification Steps

In the workflow of primary packaging entering the filling line, the de-bagger module acts as the initial up-classification step from grade CNC to grade C. Once the nests/tubs leave the de-bagger, they proceed through a second up-classification step, which may consist of an electron beam tunnel, a pulsed UV tunnel, or a similar system. Following this, the Tyvek lid is peeled off the nests/tubs, the inlay is removed, and finally, the primary packaging container is filled and closed/stoppered.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A medical component container (200) comprising:
- a container body (100) having a substantially rectangular base (102), a peripheral wall (107) having a lower end that is contiguous to a perimeter of the base (102) and extending upward, and a plurality of peripheral edges being contiguous to an upper end of the peripheral wall (107) wherein the container body (100) has an opening surrounded by the plurality of peripheral edges, and wherein the plurality of peripheral edges of the container body (100) further comprises a reinforcing frame (106) that surrounds the opening and provides structural reinforcement for the plurality of peripheral edges,
- a protective film (150) that is attached, such as by being adhered or heat-sealed, to the plurality of peripheral edges for covering and sealing the opening,
- two or more vertically stacked trays (10), each tray being substantially rectangular and each holding a plurality of medical components (1) in a regular arrangement, the lowermost tray (10) being supported on or at the base (102),
wherein the peripheral wall (107) is configured for collapsing in a manner wherein the base (102) is movable upwards towards the opening so that, after the protecting film (150) has been removed from the peripheral edges, and upon an external force acting to move the base (102) vertically upwards, the stacked trays (10) are lifted by the base (102) to emerge upwards from the plurality of peripheral edges.

2. A medical component container as in claim 1, wherein the reinforcing frame (106) is attached to the plurality of peripheral edges.

3. A medical component container as in any of claims 1 or 2, wherein the peripheral wall (107) is made of a first material having a first rigidity, and wherein the reinforcing frame (106) is made from a second material having a second rigidity greater than the first rigidity.

4. A medical component container as in claim 1, wherein the reinforcing frame (106) is formed unitarily with the peripheral wall as a thickened portion.

5. A medical component container as in any of claims 1-4, wherein the plurality of peripheral edges comprise material portions being contiguous to an upper end of the peripheral wall (107) and extend outwardly to define a contiguous flange portion (108), and wherein the protecting film (150) is adhered or heat-sealed to the contiguous flange portion (108).

6. A medical component container as in any of claims 1-5, wherein the peripheral wall (107) comprises four side walls configured as pairs of side walls arranged in opposing manner, wherein each side wall comprises a plurality of vertically stacked horizontally extending panels (107a) being interconnected by horizontally extending living hinge portions (107b).

7. A medical component container as in any of claims 1-6, wherein the peripheral wall (107) is configured to assume a corrugated shape when the container body (100) assumes a collapsed state.

8. A medical component container (200), comprising:
- a container body (100) having a substantially rectangular base (102), a peripheral wall (107) having a lower end that is contiguous to a perimeter of the base (102) and extending upward, and a plurality of peripheral edges being contiguous to an upper end of the wall (107) and extending outwardly, wherein the container body (100) has an opening surrounded by the plurality of peripheral edges, the plurality of peripheral edges defining first and second edges arranged in parallel and opposite to each other and third and fourth edges opposite to each other, the third and fourth edges extending transversely and interconnecting the first and second edges,
- a protecting film (150) having a first film section (151) that is adhered or heat-sealed to the plurality of peripheral edges for covering and sealing the opening, wherein the protecting film (150) has a second film section (152) at least partially overlapping the first film section (151), the second film section (152) having a gripping portion (155) adjacent said first edge, and being attached to the first film section (151) along said second edge so that, when the protecting film (150) is peeled open by exertion of an external pulling force (F) on the gripping portion (155) in a direction away from the second edge, the first film section (151) separates initially from the plurality of peripheral edges at a portion disposed at the second edge and progressing gradually towards the first edge.

9. A medical component container as in claim 8, wherein said external pulling force (F) is either exerted laterally away outwards from the second edge or exerted at an inclined angle relative to the lateral direction away outwards from the second edge.

10. A medical component container as in claim 8, wherein said external pulling force (F) is exerted in a direction either transverse to the first edge or generally diagonally away from the opening.

11. A medical component container as in any of claims 8-10, wherein the plurality of peripheral edges surrounding the opening of the container body (100) extend within a plane and wherein the external pulling force (F) either exerts a force in a direction within the plane or in a direction forming an angle of inclination relative to said plane, either upwards or downwards.

12. A medical component container as in any of claims 8-11, wherein the second film section (152) lies flat on top of the first film section (151).

13. A medical component container as in any of claims 8-12, wherein the second film section (12) and the first film section (151) either are made from single-sheet material and folded along the second edge, or wherein the second film section (152) is attached to the first film section (151) along said second edge.

14. A medical component container as in any of claims 8-13, wherein the first edge comprises one or more flange sections (108) contiguous to an upper end of the peripheral wall (107) and extending laterally outward therefrom, wherein the second film section (152) adheres to at least a portion of the flange sections (108), optionally via the first film section (151), towards a free, non-adhered, gripping area (155) of the second film section (152).

15. A medical component container as in any of claims 8-14, wherein the container accommodates a single tray (10) or two or more vertically stacked trays (10), each tray being substantially rectangular and each holding a plurality of medical components (1) in a regular arrangement, the lowermost tray (10) being supported on or at the base (102),
wherein the peripheral wall (107) is configured for collapsing in a manner wherein the base (102) is movable upwards towards the opening so that, after the protecting film (150) has been removed from the peripheral edges, and upon an external force acting to move the base (102) vertically upwards, the stacked trays (10) are lifted by the base (102) to emerge upwards from the plurality of peripheral edges.
